# EUROPEAN PATENT SPECIFICATION

(11) **EP 3 777 799 B1**
(45) Date of publication and mention of the grant of the patent: **14.08.2024**
(21) Application number: 19802624.7
(22) Date of filing: 09.05.2019
(51) Int. Cl.: A61F 13/51, A61F 13/514, A61F 13/84, A61F 13/49, A61F 13/495, A61F 13/53

(54) **ABSORBENT ARTICLE**
ABSORBIERENDER ARTIKEL
ARTICLE ABSORBANT

(30) Priority: 18.05.2018 JP 2018001821 U
(43) Date of publication of application: 17.02.2021
(73) Proprietor: Unicharm Corporation, Ehime 799-0111 (JP)
(72) Inventor: SHIMIZU, Noriko, Kanonji-shi, Kagawa 769-1602 (JP); MAKI, Hideaki, Kanonji-shi, Kagawa 769-1602 (JP); INOUE, Takuya, Kanonji-shi, Kagawa 769-1602 (JP)
(74) Representative: Dolleymores
(86) International application number: PCT/JP2019/018597
(87) International publication number: WO 2019/221008

(56) References cited:
- EP-A1- 3 162 342
- EP-A1- 3 318 230
- WO-A1-2017/115492
- WO-A1-2017/173645
- WO-A2-00/76439
- JP-A- 2003 501 209
- JP-A- 2007 325 915
- JP-A- 2009 538 667
- JP-A- 2010 005 342
- JP-A- 2012 105 804
- JP-A- 2014 028 308
- JP-A- 2014 028 308
- JP-A- 2016 010 517
- JP-A- 2017 012 461
- JP-A- 2018 068 898

## Description

### [Technical Field]

The present invention relates to an absorbent article.

### [Background Art]

A pull-on type of disposable diaper disclosed in Patent Document 1 includes a stomach constituent portion for covering the wearer's stomach, a back constituent portion for covering the wearer's back, and a liquid-impermeable and visually recognizable front-back identification portion (e.g., a piece of tape made of polypropylene or the like) that is affixed to either the stomach constituent portion or the back constituent portion so as to be exposed on the side opposite to the wearer's body. For this reason, the front and back of the diaper can be easily identified even if the diaper is put on in a dark place or put on by someone with a visual impairment.

### [Citation List]

### [Patent Literature]

[Patent Document 1] Japanese Patent Application Publication No. 2010-82132

JP 2014 028308 A, EP 3162342 A1 and WO 00/76439 A2 all disclose absorbent articles with graphics.

EP 3318230 A1 relates to a disposable wearing article wherein the front waist region has an appropriate fittability: an elastic strip is put in soft contact with a wearer's lower abdomen.

### [Summary of Invention]

### [Technical Problem]

However, with the diaper in Patent Document 1, the front and back of the diaper cannot be identified unless the user knows that it is predetermined that the front-back identification portion is affixed to the back constituent portion, for example. For this reason, the front and back of the diaper cannot be easily identified by a person who is not familiar with the usage of the diaper, for example.

In view of this, an aspect of the present invention is to provide an absorbent article whose front and back are likely to be identified intuitively.

### [Solution to Problem]

The present invention provides the absorbent article of independent claim 1. The dependent claims specify preferred but optional features.

### [Advantageous Effects of Invention]

According to the present invention, it is possible to provide an absorbent article whose front and back are likely to be identified intuitively.

### [Brief Description of the Drawings]

FIG. 1 is a perspective view of a pull-on disposable diaper 1.
FIG. 2 is a plan view of the diaper 1 in an unfolded and stretched state.
FIG. 3 is a cross-sectional view along a line I-I in FIG. 2.
FIG. 4 is an illustrative view of the pull-on disposable diaper 1 according to a variation.
FIG. 5 is an illustrative view of the diaper 1 according to a variation having a stool containing pocket 40.
FIG. 6 is an illustrative view of the diaper 1 according to a variation having the stool containing pocket 40.
FIG. 7 is an illustrative view of the diaper 1 according to a variation having the stool containing pocket 40.
FIG. 8 is an illustrative view of the design of the diaper 1.
FIG. 9 is an illustrative view of the design of the diaper 1.
FIG. 10 is an illustrative view of the design of the diaper 1.
FIG. 11 is an illustrative view of the design of the diaper 1.
FIG. 12 is an illustrative view of the design of the diaper 1.
FIG. 13 is an illustrative view of the design of the diaper 1.
FIG. 14 is an illustrative view of the design of the diaper 1.
FIG. 15 is an illustrative view of the design of the diaper 1.

### [Description of Embodiments]

At least the following matters will become clear with the description of this specification and the attached drawings.

An absorbent article having a vertical direction, a lateral direction, and a front-back direction that intersect each other,
the absorbent article including:
a front portion and a back portion in the front-back direction,
   the back portion including a portion having a different function from the front portion;
a front appearance graphic,
   the front appearance graphic showing at least a portion of a front appearance of a character and arranged on the front portion; and
a back appearance graphic,
   the back appearance graphic showing at least a portion of a back appearance of the character and arranged on the back portion.

According to this absorbent article, the user can intuitively identify the front and back of the absorbent article based on the front appearance graphic and the back appearance graphic.

In such an absorbent article,
the front appearance graphic is a graphic showing at least a portion of an upper body of the character, and
the back appearance graphic is a graphic showing at least a portion of a lower body of the character.

According to this absorbent article, a characteristic portion of the front appearance of the character is shown by the front appearance graphic, and a characteristic portion of the back appearance of the character is shown by the back appearance graphic. For this reason, the user can more intuitively identify the front and back of the absorbent article.

In such an absorbent article,
an area of the back appearance graphic is larger than an area of the front appearance graphic.

According to this absorbent article, the back portion is more prominent than the front portion, thus making it possible to give the user the impression (illusion) that the back portion is large, and allowing the user to more intuitively identify the front and back of the absorbent article.

In such an absorbent article,
an upper end of the back appearance graphic is located higher than an upper end of the front appearance graphic, and
a lower end of the back appearance graphic is located lower than a lower end of the front appearance graphic.

According to this absorbent article, the size of the back appearance graphic can be increased in the vertical direction.

In such an absorbent article,
the front portion has text indicating that it is a portion that is to come into contact with a front side of a wearer.

According to this absorbent article, a user who sees the front appearance graphic for the first time can immediately identify the front and back of the absorbent article.

In such an absorbent article,
the back portion does not have text indicating that it is a portion that is to come into contact with a back side of a wearer.

According to this absorbent article, the size of the back appearance graphic can be commensurately increased due to not providing text. Also, a user who sees the back appearance graphic can intuitively identify the front and back of the absorbent article even if such text is not provided.

The absorbent article of the present invention is an underpants-shaped absorbent article,
the underpants-shaped absorbent article including a front waist region, a back waist region, and a crotch region,
two lateral side end portions of the front waist region being joined to two lateral side end portions of the back waist region,
the front waist region and the back waist region each including a stretchable member capable of stretching and contracting in the lateral direction.

Accordingly, even if the absorbent article is an underpants-shaped absorbent article whose front and back are difficult to identify, the user can intuitively identify the front and back of the absorbent article.

In the absorbent article of the present invention,
a length of a stretchable portion of a stretchable member that is overlapped with the front appearance graphic is smaller than a length of a stretchable portion of a stretchable member that is overlapped with the back appearance graphic.

Accordingly, it is possible to suppress the case where small parts (e.g., ears, eyes, and nose) of the character
shown by the front appearance graphic are difficult to see due to contraction of the stretchable member. On the other hand, the size of the back appearance graphic can be increased.

In such an absorbent article,
text indicating that it is a portion that is to come into contact with either one of a front side of a wearer and a back side of a wearer is arranged in the crotch region.

According to this absorbent article, it is possible to suppress the case where the text is difficult to be visually recognized due to contraction of the stretchable member.

In such an absorbent article,
the back appearance graphic is arranged extending from the back waist region into the crotch region.

According to this absorbent article, the back appearance graphic is large, and therefore the back appearance graphic is more likely to be recognized.

In such an absorbent article,
an area of a portion of the back appearance graphic arranged in the back waist region is larger than an area of a portion of the back appearance graphic arranged in the crotch region.

According to this absorbent article, the stretchable member arranged in the back waist region is more likely to be recognized by the user.

In such an absorbent article,
the front appearance graphic is arranged extending from the front waist region into the crotch region.

According to this absorbent article, the front appearance graphic is large, and therefore the front appearance graphic is more likely to be recognized.

In such an absorbent article,
a value obtained by dividing an area of a portion of the back appearance graphic arranged in the crotch region by a total area of the back appearance graphic
   is greater than
a value obtained by dividing an area of a portion of the front appearance graphic arranged in the crotch region by a total area of the front appearance graphic.

According to this absorbent article, the back portion is more prominent than the front portion, thus making it possible to give the user the impression (illusion) that the back portion is large, and allowing the user to more intuitively identify the front and back of the absorbent article.

In such an absorbent article,
post-processing tape is not provided in a lateral central portion of the back waist region.

According to this absorbent article, even if the absorbent article is one whose front and back are difficult to identify, the user can intuitively identify the front and back of the absorbent article.

In such an absorbent article,
the front waist region is provided with a stretchy sheet as the stretchable member, and
the back waist region is not provided with the stretchy sheet.

According to this absorbent article, the front waist region can come into surface-to-surface contact with the wearer's stomach region, and it is possible to suppress constriction of the wearer's stomach region.

In such an absorbent article,
the absorbent article further comprises a front waist region, a back waist region, and a crotch region,
the back waist region is provided with a pocket,
   the pocket being located on a non-skin side with respect to a waist contact portion extending in the lateral direction,
   the pocket facing the waist contact portion in the front-back direction at a lateral central portion,
   the pocket being capable of opening toward the crotch region, and
the front waist region is not provided with the pocket.

According to this absorbent article, loose stool can be contained in the pocket, and it is possible to suppress the leakage of loose stool on the back side.

In such an absorbent article,
the absorbent article further comprises an absorbent core,
the absorbent core includes a narrow portion,
   the narrow portion having a lateral width that is smaller than a lateral width of an upper end of the absorbent core, and
a vertical length of a portion of the narrow portion arranged in the front portion is larger than a vertical length of a portion of the narrow portion arranged in the back portion.

According to this absorbent article, the absorbent core fits to the wearer's body, and it is possible to suppress the leakage of excrement.

In such an absorbent article,
the back portion is provided with a sweat absorbing sheet containing hydrophilic fibers, on a skin-side surface of the back portion, and
the front portion is not provided with the sweat absorbing sheet.

According to this absorbent article, sweat on the wearer's back side can be absorbed, and it is possible to suppress rashes and the like.

### Embodiments

The following describes an embodiment of an absorbent article according to the present invention by way of example of a pull-on disposable diaper for an infant. However, the absorbent article of the present invention is not limited to being a pull-on disposable diaper for an infant, and is also applicable to a pull-on disposable diaper for an adult, a tape-type disposable diaper, sanitary shorts, and the like.

### Configuration of pull-on disposable diaper 1

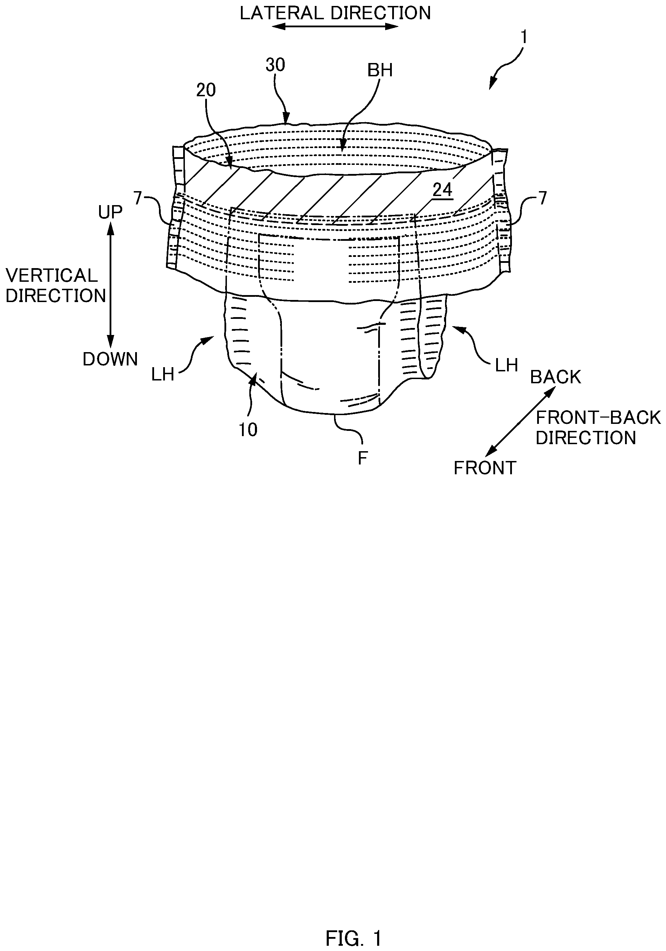

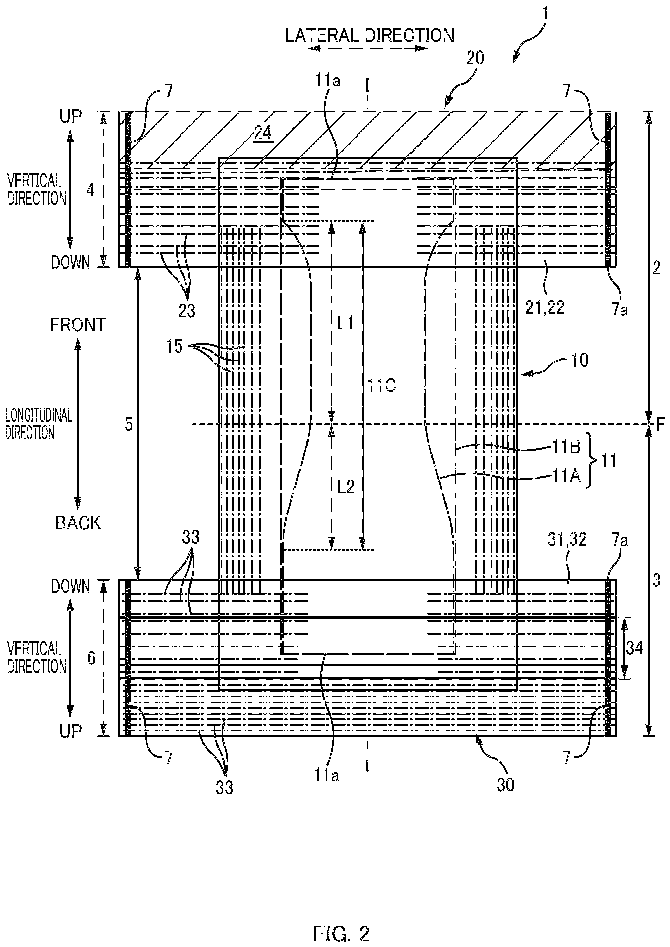

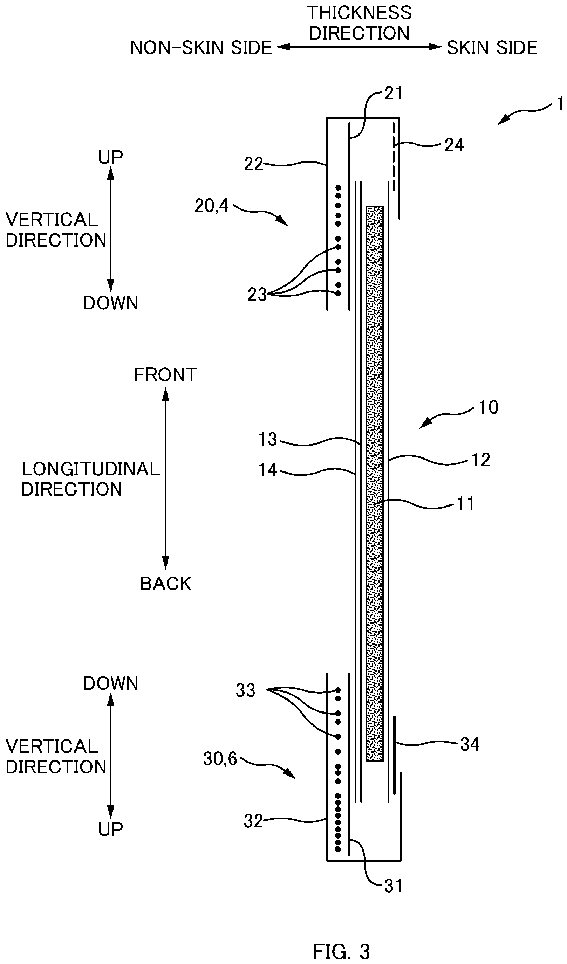

FIG. 1 is a perspective view of a pull-on disposable diaper (hereinafter called "diaper") 1. FIG. 2 is a plan view of the diaper 1 in an unfolded and stretched state. FIG. 3 is a cross-sectional view along a line I-I in FIG. 2.

In the underpants-shaped state shown in FIG. 1, the diaper 1 has a vertical direction, a lateral direction, and a front-back direction that intersect each other, and includes a waist opening BH and a pair of leg openings LH. In the vertical direction, the waist side is the upper side, and the crotch side is the lower side. In the front-back direction, the wearer's stomach side is the front side, and the wearer's back side is the back side. Also, as shown in FIG. 3, the direction in which constituent members of the diaper 1 are overlaid on each other is the thickness direction, and in the thickness direction, the side that comes into contact with the wearer is the skin side, and the side that does not come into contact with the wearer is the non-skin side.

Also, the diaper 1 includes an absorbent main body 10 and a pair of belt members 20 and 30, which are rectangular in a plan view. Out of the two belt members 20 and 30, the belt member that comes into contact with the wearer's stomach-side region is the front belt member 20, and the belt member that comes into contact with the wearer's back-side region is the back belt member 30.

In the diaper 1 in the unfolded state shown in FIG. 2, the front belt member 20 and the back belt member 30 are arranged such that their longitudinal direction matches the lateral direction of the diaper 1. The longitudinal one-side end portion of the absorbent main body 10 is arranged at the lateral central portion of the front belt member 20, and the longitudinal other-side end portion of the absorbent main body 10 is arranged at the lateral central portion of the back belt member 30.

In the diaper 1 in the unfolded state shown in FIG. 2, the absorbent main body 10 is folded one time at an approximate center F in the longitudinal direction such that the longitudinal direction of the absorbent main body 10 matches the vertical direction of the diaper 1, and the two lateral end portions of each of the front belt member 20 and the back belt member 30 are joined to each other to form a pair of joining portions 7, thus obtaining the diaper 1 in the underpants-shaped state shown in FIG. 1. Note that welding and joining by an adhesive or the like are examples of joining methods used for the joining portions 7.

As shown in FIG. 3, the absorbent main body 10 includes an absorbent body 11, a liquid-permeable top sheet 12 arranged on the skin side of the absorbent body 11, a liquid-impermeable back sheet 13 arranged on the non-skin side of the absorbent body 11, and an exterior sheet 14 arranged on the non-skin side of the back sheet 13. The exterior sheet 14 is preferably a soft sheet. Also, leg elastic members 15 are provided in the two lateral side portions of the absorbent main body 10, thus allowing the diaper 1 to fit around the wearer's legs.

As shown in FIG. 2, the absorbent body 11 includes an absorbent core 11A that absorbs and holds an excreted fluid such as urine, and a liquid-permeable core-wrapping sheet 11B that covers the absorbent core 11A. As one example of the absorbent core 11A, liquid-absorbent fibers such as pulp containing a superabsorbent polymer (SAP) are shaped into a predetermined shape. Note that the absorbent core 11A is not required to be covered by the core-wrapping sheet 11B.

As shown in FIG. 3, the front belt member 20 (the back belt member 30) includes a skin-side sheet 21 (31), a non-skin-side sheet 22 (32), and elastic strings (stretchable members) 23 (33) that stretch and contract in the lateral direction. The elastic strings 23 (33) are arranged side-by-side in the vertical direction between the skin-side sheet 21 (31) and the non-skin-side sheet 22 (32), and are fixed in a state of being stretched in the lateral direction. Accordingly, the front belt member 20 and the back belt member 30 stretch and contract in the lateral direction so as to fit around the wearer's waist.

In the following description, the portion of the front belt member 20 overlapped with the joining portions 7 in the vertical direction is called a "front waist region 4", the portion of the back belt member 30 overlapped with the joining portions 7 in the vertical direction is called a "back waist region 6", and the portion between the front waist region 4 and the back waist region 6 (i.e., the portion between lower ends 7a of the joining portions 7) is called a "crotch region 5". In the case of the diaper 1 shown in FIG. 2, the entirety of the front belt member 20 is in the front waist region 4, and the entirety of the back belt member 30 is in the back waist region 5.

Also, the diaper 1 shown in FIG. 2 is a so-called three-piece type of diaper that includes the front belt member 20 and the back belt member 30, which are separate from each other, as well as the absorbent main body 10. Although not shown, the diaper 1 may be a so-called twopiece type of pull-on disposable diaper that includes two members, namely an absorbent main body and an exterior member in which a front belt member and a back belt member are connected by a crotch member. Similarly, in this case as well, the crotch region is the portion between the lower ends of joining portions that join the front waist region and the back waist region, and the front waist region and the back waist region are the portions overlapped with the joining portions in the vertical direction.

### Differences in functions of front portion 2 and back portion 3

In the diaper 1 in the underpants-shaped state, the portion forward with respect to a fold F where the absorbent main body 10 is folded (i.e., a lower end F of the diaper 1 in the underpants-shaped state) is a "front portion 2", and the portion backward with respect to the fold F is a "back portion 3". The diaper 1 of the present embodiment has an asymmetrical shape in the front-back direction. The front portion 2 and the back portion 3 have portions that have different structures, and these different-structures portions have different functions.

Note that packaged tape-type diapers (not shown) are generally folded at the approximate center in the product longitudinal direction, with the skin-side surface on the inward side. Accordingly, in the case of a tape-type diaper, the portion forward of the fold made during packaging is the front portion, and the portion backward of the fold made during packaging is the back portion.

The following describes specific examples of portions of the front portion 2 and the back portion 3 that have different functions. It is preferable that the diaper 1 includes one or more of the following examples of portions that have different functions. Note that there is no limitation to the examples of portions described below, and the front portion 2 and the back portion 3 may include other portions that have different functions.

### Different portions: first example

As shown in FIGS. 2 and 3, the back waist region 6 of the diaper 1 is provided with elastic strings 33 arranged side-by-side from the upper end portion to the lower end portion. On the other hand, the upper end portion of the front waist region 4 is provided with a stretchy sheet 24 instead of elastic strings, as a stretchable member that stretches and contracts in the lateral direction.

In this way, the back waist region 6 is not provided with a stretchy sheet, whereas the front waist region 4 is provided with the stretchy sheet 24, and therefore the upper end portion of the front waist region 4 comes into surface-to-surface contact with wearer. For this reason, localized constriction is suppressed, and the stretchable member is not likely to form marks on the wearer's skin. In particular, infant's stomachs bulge outward, and therefore it is preferable to suppress constriction by the front waist region 4. On the other hand, due to the elastic strings 33, the back waist region 6 can come into close contact with the wearer's back side, and it is possible to suppress the leakage of loose stool or the like on the back side.

Note that examples of the stretchy sheet 24 includes: a stretchable film sheet made of plastic; non-woven fabric that has been subjected to stretch processing such as gear stretching and includes elastic thermoplastic elastomer fibers made of a polyurethane elastomer and non-elastic thermoplastic resin fibers made of a polyolefin resin such as polypropylene (PP); or the like.

### Different portions: second example

Although the front waist region 4 and the back waist region 6 are both provided with a stretchable member (elastic strings or a stretchy sheet) that stretches and contracts in the lateral direction, it is desirable that the lateral stretching force per vertical unit width is different between them. Specifically, it is desirable that a value obtained when the amount of force required to stretch the waist regions 4 and 6 in the lateral direction by a unit length is divided by the vertical length of the waist regions 4 and 6 is different between the waist regions. In the present embodiment, the stretching force of the front waist region 4 is set smaller than the stretching force of the back waist region 6. Accordingly, the lower stretching force of the front waist region 4 suppresses constriction of a bulging stomach region, and the higher stretching force of the back waist region 6 can suppress the leakage of loose stool or the like on the back side.

Note that the stretching force can be lowered by, for example, lowering the fiber density (reducing the diameter) of the elastic strings, lowering the stretch factor of the stretchable member, shortening the effective length of the stretchable member, reducing the number of elastic strings per vertical unit width, or shortening the vertical width of the stretchy sheet. The stretching force can be raised by making opposite adjustments. The stretch factor of the stretchable member is the extent of stretching relative to a natural length of 1 for the stretchable member. The effective length of the stretchable member is the length of the portion of the stretchable member that exhibits stretchability, and is the length of the portions where the stretchable member is fixed in the stretched state to a sheet.

Also, a known method can be used to measure the amount of force required to stretch the waist regions 4 and 6 in the lateral direction by a unit length. For example, measurement can be performed using a tension tester such as Autograph (AGS-G100N) made by Shimadzu Corporation.

### Different portions: third example

The wearer of the diaper 1 may have a rash or the like on their back caused by sweat. In view of this, as shown in FIGS. 2 and 3, it is desirable that the front portion 2 is not provided with a sweat absorbing sheet, and the back portion 3 is provided with a sweat absorbing sheet 34 on the skin-side surface.

The sweat absorbing sheet 34 is a sheet that easily absorbs sweat, and includes hydrophilic fibers (water absorbing fibers). Preferably, the hydrophilic fibers are provided with a medicinal component that has an effect of resisting or killing bacteria that causes rashes or the like. As one specific example, the sweat absorbing sheet 34 has a skin-side layer that is mainly composed of hydrophobic fibers and includes few hydrophilic fibers, and a non-skin-side layer that is mainly composed of hydrophilic fibers. When using this sweat absorbing sheet 34, sweat is absorbed by the hydrophilic fibers in the skin-side layer, and the absorbed sweat can permeate into and spread in the non-skin-side layer. Also, the effusion of sweat can be suppressed by the hydrophobic fibers in the skin-side layer. This therefore eliminates a sense of moistness where sweating occurs, and achieves a cool sensation. Accordingly, the comfort of the diaper 1 (back portion 3) improves, and rashes and the like can be suppressed. On the other hand, the front portion 2 is not provided with a sweat absorbing sheet, thus reducing the cost of the diaper 1.

### Different portions: fourth example

As shown in FIG. 2, the absorbent core 11 includes a narrow portion 11C whose width in the lateral direction is smaller than that of an upper end 11a of the absorbent core 11. A vertical length L1 of the portion of the narrow portion 11C arranged in the front portion 2 is larger than a vertical length L2 of the portion of the narrow portion 11C arranged in the back portion 3. In other words, it is desirable that the narrow portion 11C has an asymmetrical shape in the front-back direction, and is arranged eccentric toward the front portion 2.

This is because infants, who are the target wearer of the diaper 1, often move their legs on the front side when laying face-up or crawling. For this reason, the narrow portion 11C, which is eccentric toward the front portion 2, is sandwiched between the wearer's legs, and the absorbent core 2 fits to the wearer's body. Also, excrement (urine or feces) can be stopped by the wide portions of the absorbent core 2 on the two vertical sides of the narrow portion 11C, making it possible to suppress the leakage of excrement.

### Different portions: fifth example

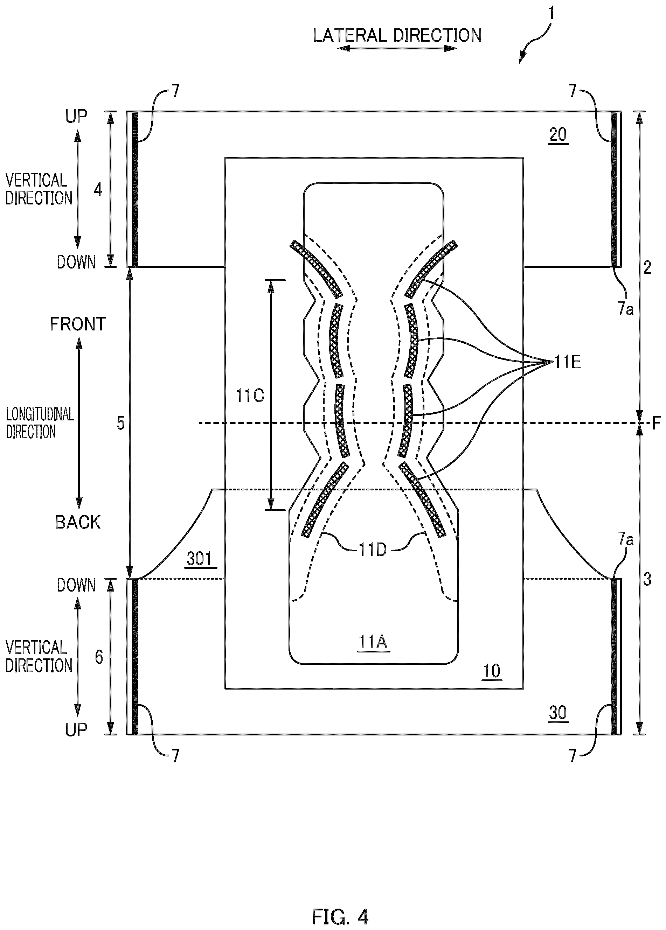

FIG. 4 is an illustrative view of the pull-on disposable diaper 1 according to a variation, and is a schematic plan view of the diaper 1 in an unfolded and stretched state. The absorbent core 11A of the diaper 1 shown in FIG. 4 is provided with low-basis-weight portions 11D. The low-basis-weight portions 11D are portions where the basis weight (mass per unit area [g/m²]) of the absorbent core 11A is lower than the surrounding region. Also, along the lo........w-basis-weight portions 11D, provided are compressed portions 11E (embossed portions), in which at least a portion of the absorbent core 11A is compressed in the thickness direction.

The low-basis-weight portions 11D and the compressed portions 11E extend along the side ends of the narrow portion 11C of the absorbent core 11A. More specifically, the low-basis-weight portions 11D and the compressed portions 11E include portions that are approximately parallel with the longitudinal direction in the longitudinal central portion of the narrow portion 11C, and portions that extend outward from the those portions toward the two longitudinal sides. The absorbent core 11A can easily fold at the low-basis-weight portions 11D and the compressed portions 11E. Also, similarly to the narrow portion 11C described in the fourth example, it is desirable that the low-basis-weight portions 11D and the compressed portions 11E have an asymmetrical shape in the front-back direction and are arranged eccentric toward the front portion 2. Accordingly, in the longitudinal central portion of the narrow portion 11C, the absorbent core 11A can easily fold when being sandwiched between the wearer's legs, which tend to be located on the forward side, thus achieving a good fit to the wearer's body. On the other hand, the two vertical sides of the narrow portion 11C are not likely to be subjected to the bending of the absorbent core 11A, thus maintaining a wide shape and making it possible to stop the movement of excrement.

Additionally, because the urination position is eccentric toward the front portion 2 side, it is desirable that the basis weight of the portion of the absorbent core 11A in the front portion 2 is higher than the basis weight of the portion of the absorbent core in the back portion 3. In other words, the distribution of the basis weight of the absorbent core 11A may be set differently in the front portion 2 and the back portion 3.

### Different portions: sixth example

Although the front belt member 20 and the back belt member 30 of the diaper 1 shown in FIG. 2 have the same shape, there is no limitation to this. As shown in FIG. 4, the back belt member 30 may have an extension portion 301 that extends downward from the back waist region 6. In other words, the belt members 20 and 30 may have different shapes in the front portion 2 and the back portion 3. The wearer's buttocks region can be covered by the extension portion 301 of the back belt member 30. On the other hand, due to not providing an extension portion in the front belt member 20, the wearer can easily move their legs on the forward side.

Note that even in the case of the diaper shown in FIG. 4, the portion between the lower ends 7a of the joining portions 7 that join the front waist region 4 and the back waist region 6 is the crotch region, and the portions overlapped in the vertical direction with the joining portions 7 are the front waist region 4 and the back waist region 6.

### Different portions: seventh example

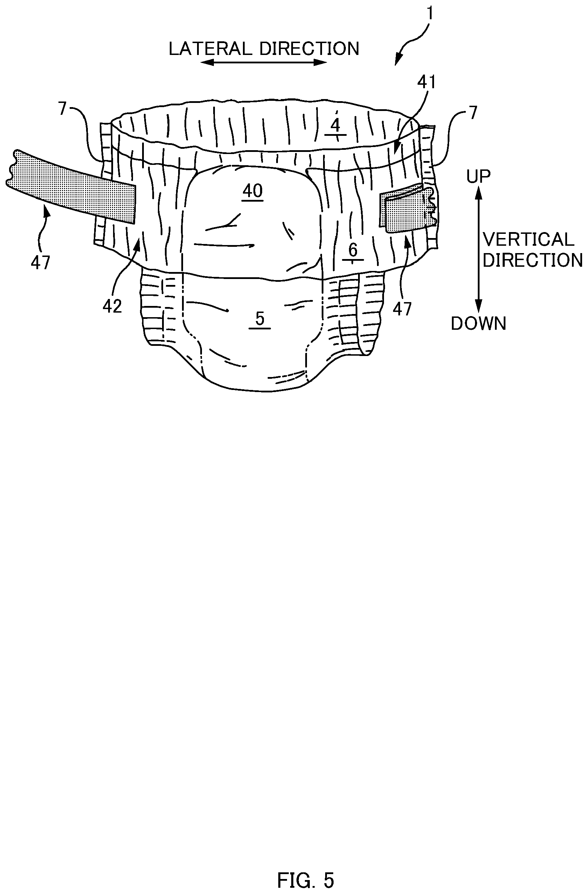

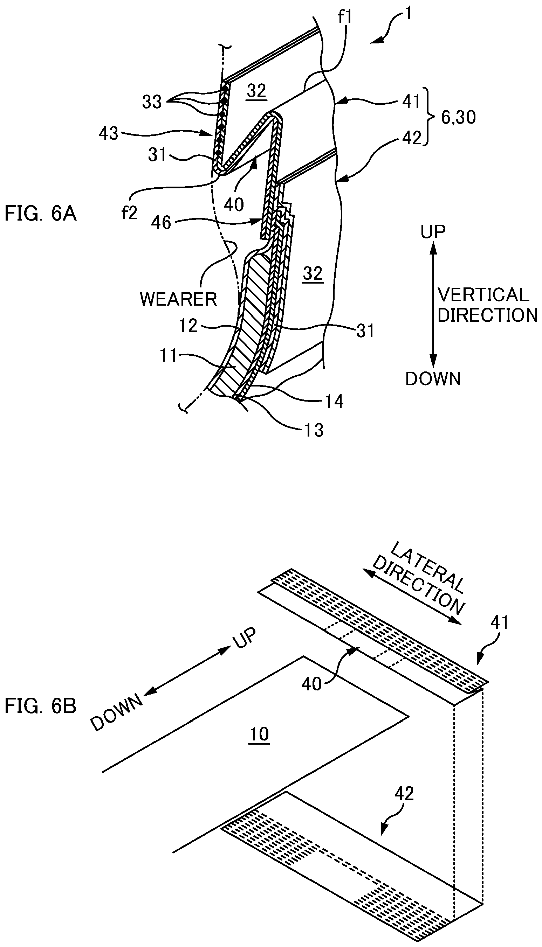

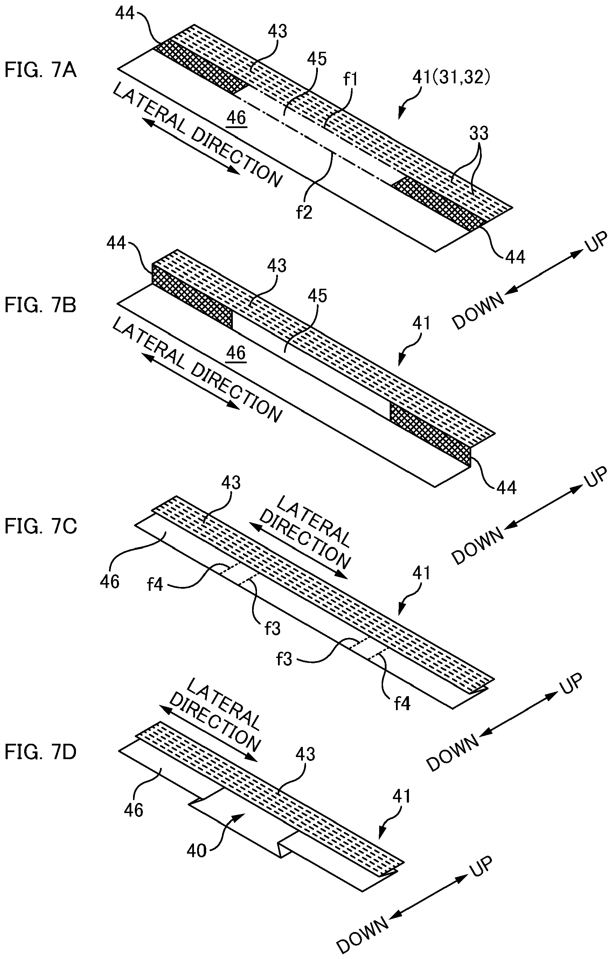

FIGS. 5 to 7 are illustrative views of the diaper 1 according to a variation having a stool containing pocket 40. FIG. 5 is a perspective view of the diaper 1 from the back side. FIG. 6A is a cross-sectional view of the diaper 1 on the back waist region 6 (back belt member 30) side. FIG. 6B is a diagram illustrating the configuration of the back waist region 6. FIGS. 7A to 7D are diagrams showing the process of folding an upper end member 41 of the back waist region 6.

As shown in FIG. 6A, the back waist region 6 may be provided with a stool containing pocket 40 that is located on the non-skin side with respect to a waist contact portion 43 extending in the lateral direction. The stool containing pocket 40 faces the waist contact portion 43 in the front-back direction at its lateral central portion and can open toward the crotch region. Accordingly, loose stool, which is characteristic of infants up to several months old, can be contained in the stool containing pocket 40, and it is possible to prevent the leakage of excrement on the back side. On the other hand, loose stool is not likely to flow on the front side, and therefore it is preferable that the front waist region 4 is not provided with a stool containing pocket.

As shown in FIG. 6B, the back waist region 6 that includes the stool containing pocket 40 is formed as follow: an upper end member 41 is overlaid on the skin side of the upper end portion of the absorbent main body 10, and a lower end member 42 is overlaid on the non-skin side of the upper end portion of the absorbent main body 10. The upper end member 41 and the lower end member 42 are members where the elastic strings 33 are fixed between the skin-side sheet 31 and the non-skin-side sheet 32, similarly to the back waist region 6 (the back belt member 30) shown in FIG. 3.

However, as shown in FIGS. 7A and 7B, the upper end member 41 is folded at folding lines f1 and f2 that extend in the lateral direction, such that the upper end portion 43 (waist contact portion 43) of the upper end member 41 is overlaid on a lower end portion 46. Also, two lateral end portions 44 of the portion between the folding lines f1 and f2 are fixed to the lower end portion 46 using an adhesive or the like, and a lateral central portion 45 is not fixed to the lower end portion 46, so as to be able to move away from the lower end portion 46. Furthermore, as shown in FIGS. 7C and 7D, the two lateral side portions of the portion of the lower end portion 46 not joined to the intermediate portion 45 are folded at a pair of folding lines f3 and f4 that extend in the vertical direction. Accordingly, in the lateral central portion of the upper end member 41, the lower end portion 46 opposes the upper end portion 43 (waist contact portion 43) in the front-back direction and can three-dimensionally move away to the non-skin side, thus forming the stool containing pocket 40. The stool containing pocket 40 can open toward the crotch side at the folding line f1, and a space that is closed toward the waist side is formed by the folding line f2.

The stool containing pocket 40 described above is one example, and there is no limitation to this. For example, the upper end member 41 and the lower end member 42 may be integrated as a single member, and the lower end portion 46 of the upper end member 41 may be folded at the folding lines f3 and f4 as shown in FIG. 7C.

### Design of front portion 2 and back portion 3

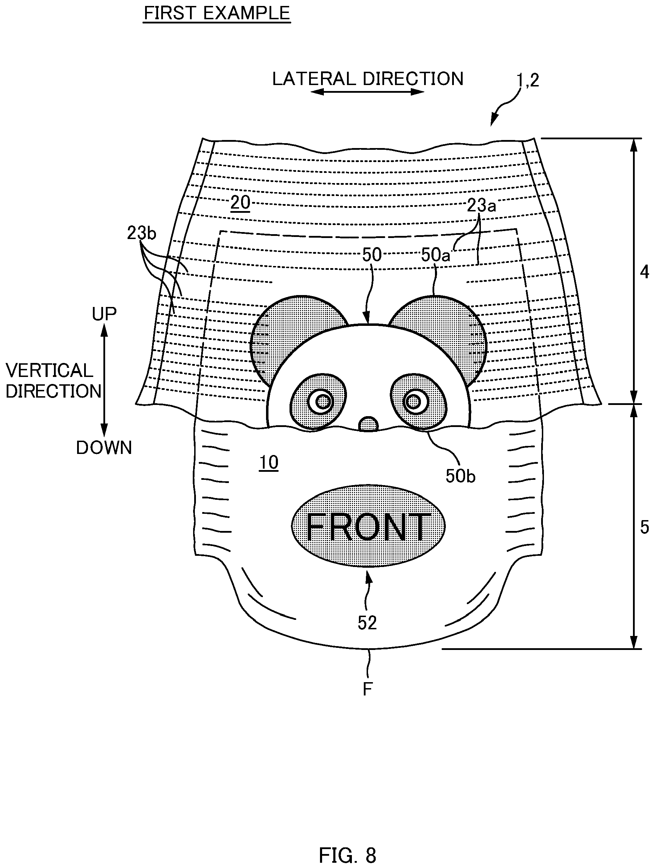

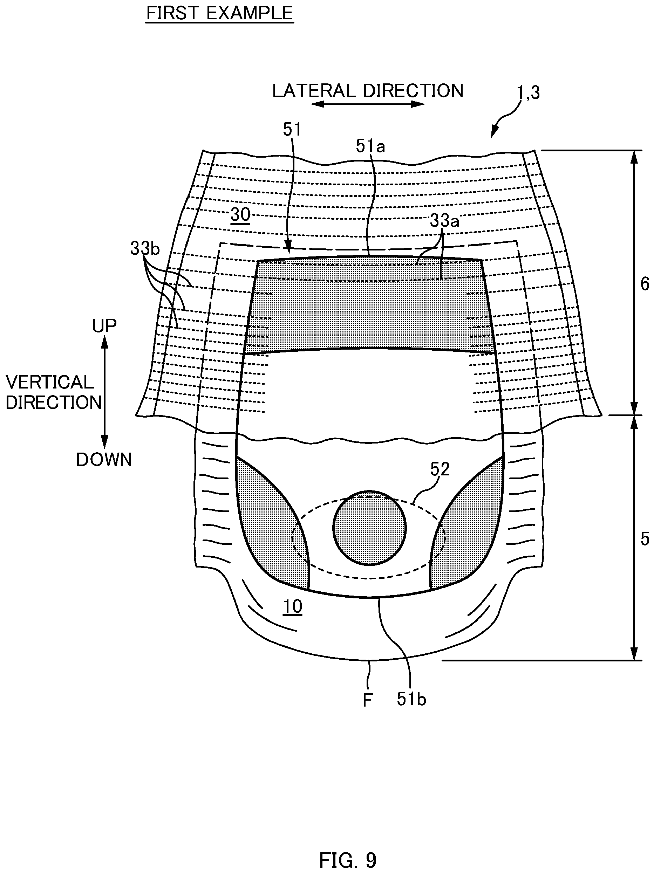

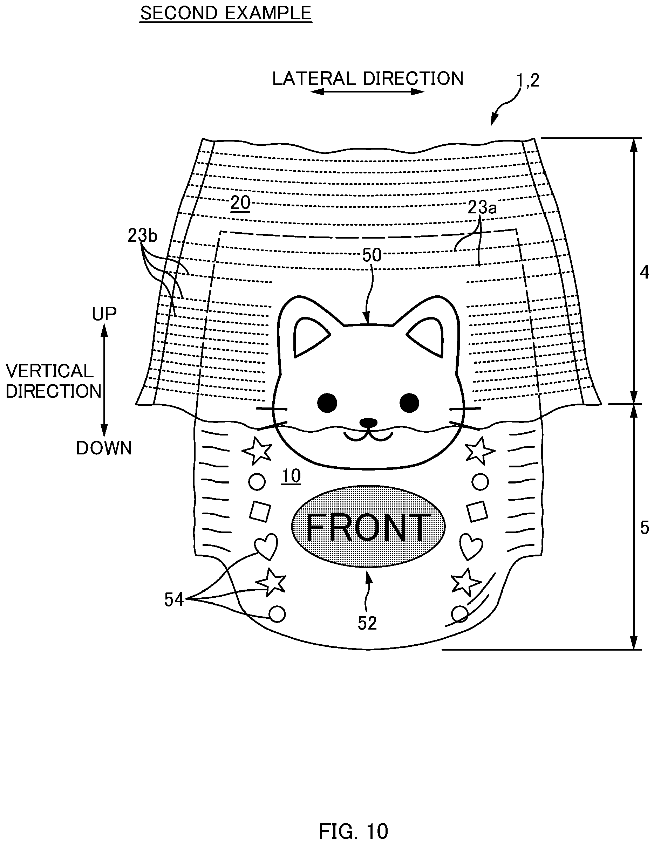

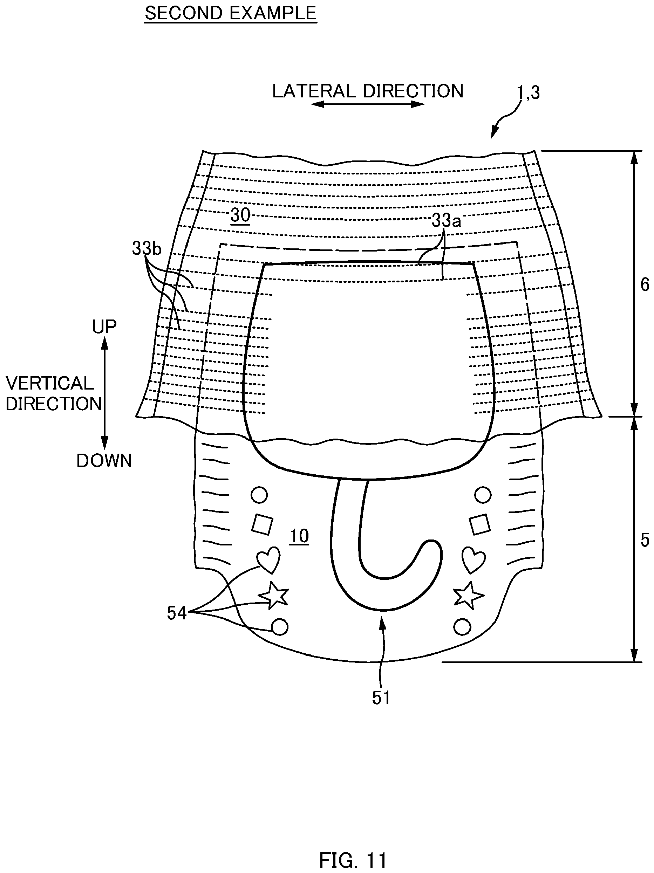

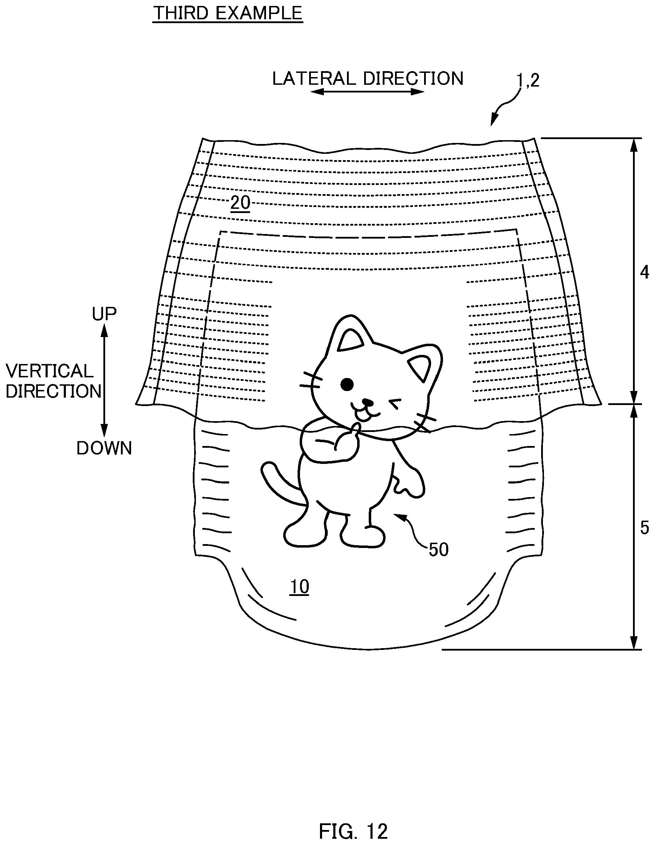

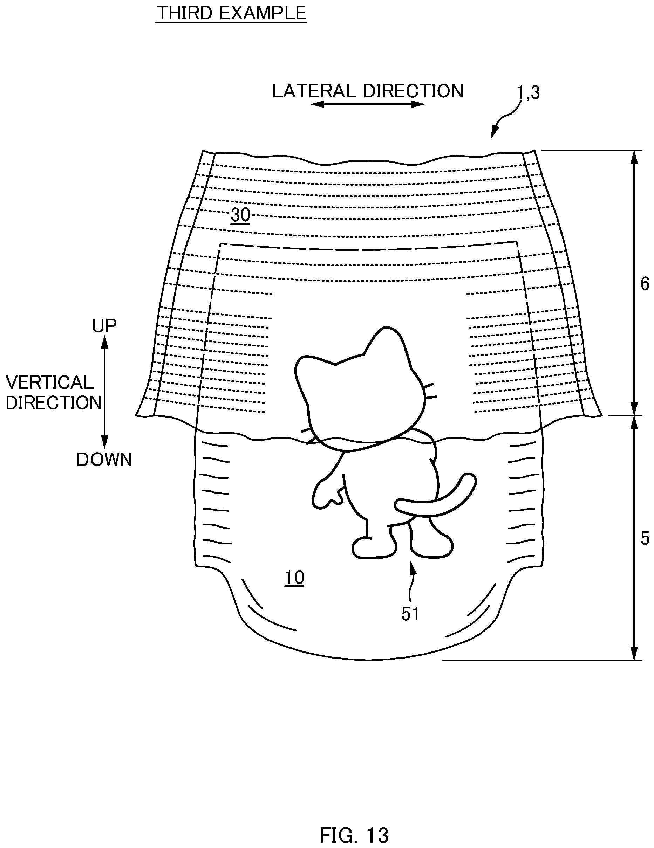

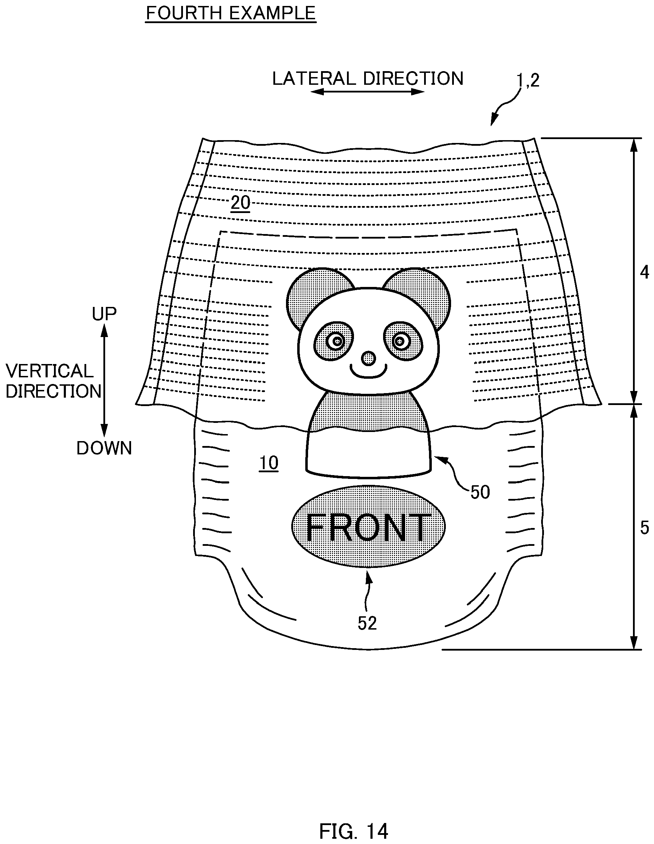

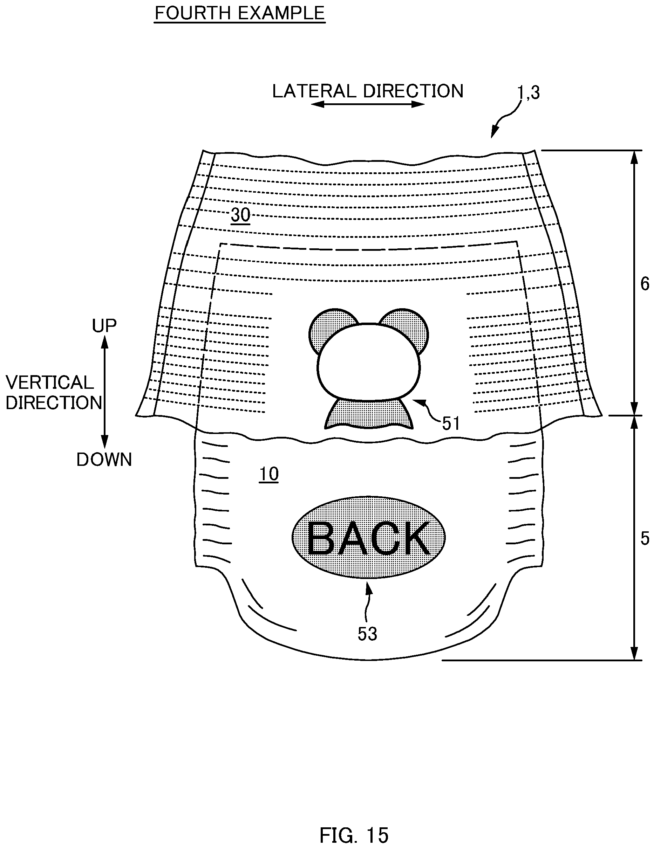

FIGS. 8 to 15 are illustrative views of designs of the diaper 1. FIGS. 8 and 9 show a diaper 1 according to a first example, FIGS. 10 and 11 show the diaper 1 according to a second example, FIGS. 12 and 13 show the diaper 1 according to a third example, and FIGS. 14 and 15 show the diaper 1 according to a fourth example. Also, FIGS. 8, 10, 12, and 14 show designs of the front portion 2, and FIGS. 9, 11, 13, and 15 show designs of the back portion 3.

As previously described, the front portion 2 and the back portion 3 of the diaper 1 include portions that have mutually different functions. For this reason, if the diaper 1 is put on backwards in the front-back direction, the functionality of the diaper 1 according to the differences between the front and back sides of the wearer is no longer exhibited.

In view of this, in the diaper 1 of the present embodiment, a front appearance graphic 50 showing at least a portion of the front appearance of a character is arranged on the front portion 2, and a back appearance graphic 51 showing at least a portion of the back appearance of the character is arranged on the back portion 3. For example, in the case of the diaper 1 according to the first example, part of the face of a panda (character) is printed as the front appearance graphic 50 on the front portion 2 (FIG. 8), and the panda's (character's) back and buttocks region and tail are printed as the back appearance graphic 51 on the back portion 3 (FIG. 9).

According to this configuration, the user of the diaper 1 (wearer or caregiver (wearer's guardian etc.)) can intuitively identify the front and back of the diaper 1 by viewing the front appearance graphic 50 and the back appearance graphic 51. Accordingly, it is possible to suppress the case where the diaper 1 is put on backwards in the front-back direction, and the functionality of the diaper 1 according to the differences between the front and back sides of the wearer is exhibited. Also, the diaper 1 can be replaced smoothly. Furthermore, even if the diaper is put on backwards, the mistake can be realized immediately. Moreover, if wrinkling or bunching occurs in the diaper 1, the front appearance graphic 50 and the back appearance graphic 51 are more easily recognized than text such as "front" and "back". For this reason, due to making it possible to identify the front and back based on the front appearance graphic 50 and the back appearance graphic 51, the front and back are more likely to be recognized even if wrinkling or bunching occurs in the diaper 1.

In the case of a tape-type diaper (not shown), a pair of pieces of fastening tape extend from the two side end portions of the back waist region. For this reason, the user can identify the front and back of the diaper based on the pieces of fastening tape. However, because the pull-on disposable diaper 1 does not include pieces of fastening tape, the front and back of the diaper 1 are more difficult to identify than in the case of a tape-type diaper. In particular, if the front belt member 20 and the back belt member 30 have the same shape as shown in FIGS. 1 and 2, the front and back of the diaper 1 are even more difficult to identify. For this reason, it can be said to be more effective if the front appearance graphic 50 and the back appearance graphic 51 are arranged on the pull-on disposable diaper 1 whose front and back are difficult to identify.

It should be noted that pull-on disposable diapers are often provided with a piece of post-processing tape (not shown) on the lateral central portion of the back waist region 6. The post-processing tape is tape for keeping the diaper in a rolled-up state when the diaper is discarded. The post-processing tape is elongated in the vertical direction of the diaper and folded into a Z-like shape.

A user familiar with the usage of pull-on disposable diapers can identify the front and back of the diaper based on the post-processing tape. However, the post-processing tape is a small, transparent member, and is inconspicuous due to blending in with graphics provided on the back waist region 6. For this reason, at nighttime or in a dimly-lit space such as a toilet stall, the post-processing tape is difficult to find, and the front and back of the diaper cannot be identified immediately. In contrast, the front appearance graphic 50 and the back appearance graphic 51 are more prominent than post-processing tape, and the front and back of the diaper 1 can be immediately identified in even a dimly-lit space.

Also, with the pull-on disposable diaper 1 that includes the above-described stool containing pocket 40 (FIG. 5) for example, there are cases where the post-processing tape is not provided on the lateral central portion of the back waist region 6. With the diaper 1 shown in FIG. 5, a pair of pieces of post-processing tape 47, which are elongated in the lateral direction and folded in a Z-like shape, are provided on the two lateral end portions (near the joining portions 7) of the back waist region 6. However, the pair of pieces of post-processing tape 47 provided near the joining portions 7 are less recognizable than a piece of post-processing tape provided on the central portion of the back waist region 6, and are not likely serve as a basis for identifying the front and back of the diaper 1. Also, in the case of the pieces of post-processing tape 47 provided near the joining portions 7, whether they are affixed to the waist region 4 or 6 cannot be easily identified at a glance. For this reason, it can be said to be more effective to provide the front appearance graphic 50 and the back appearance graphic 51 on the pull-on disposable diaper 1 that does not include post-processing tape on the lateral central portion of the back waist region 6.

There are also cases where the diaper 1 is replaced by a user who is not familiar with the usage of the diaper 1, such as the infant's grandmother or grandfather. A user who is not familiar with the usage of the diaper 1 often will not know that it is predetermined that the side of a tape-type diaper that has the fastening tape is the back side, or the side of a pull-on disposable diaper that has the post-processing tape is the back side, for example. For this reason, if the front appearance graphic 50 and the back appearance graphic 51 are provided on both a tape-type diaper and the pull-on disposable diaper 1, the front and back of the diaper can be intuitively identified by even a user who is not familiar with the usage of the diaper 1.

Text or graphics such as the front appearance graphic 50 and the back appearance graphic 51 are printed on the non-skin-side surface of the liquid-impermeable back sheet 13 of the absorbent main body 10.

Also, the characters serving as the graphics need only be able to be identified as a front appearance and a back appearance by the user of the diaper 1. Specific examples of characters include an animal such as the illustrated panda or a cat, a person, a robot, and an imaginary creature. Also, it is desirable that the front appearance graphic 50 and the back appearance graphic 51 depict the same character on the front portion 2 and the back portion 3 of the same diaper 1.

Also, it is desirable that the front appearance graphic 50 is a graphic that shows at least a portion of the upper body of a character, and the back appearance graphic 51 is a graphic that shows at least a portion of the lower body of the character, as with the diaper 1 according to the first example and the second example. The upper body of a character is the portion from the character's waist to its head. The lower body of a character is the portion from the character's waist to its buttocks. Specifically, it is desirable that the front appearance graphic 50 is a graphic that shows the face, ears, and hands of a character, and the back appearance graphic 51 is a graphic that shows the buttocks, tail, and legs of the character.

In the case of a character's front appearance, the upper body is more characteristic than the lower body, and in the case of a character's back appearance, the lower body is more characteristic than the upper body. For this reason, based on the front appearance graphic 50 and the back appearance graphic 51, the user can more intuitively identify the front and back of the diaper 1.

Note that as with the diaper 1 according to a third example, the front appearance graphic 50 may show the front appearance of the entire body of a character (cat), and the back appearance graphic 51 may show the back appearance of the entire body of the character (cat). Also, as with the diaper 1 according to a fourth example, the back appearance graphic 51 may show the back of the character's head.

However, if the front appearance graphic 50 and the back appearance graphic 51 show parts of the character that are characteristic to the front appearance and the back appearance respectively, such graphics can show larger views of those portions of the character. Specifically, the cat's face in the front appearance graphic 50 in the second example can be larger than the cat's face in the front appearance graphic 50 in the third example, and the cat's buttocks and tail in the back appearance graphic 51 in the second example can be larger than the cat's buttocks and tail in the back appearance graphic 51 in the third example. Accordingly, even if the diaper 1 is replaced by in a dimly-lit place or the diaper 1 is replaced by an elderly person (grandparent) who has difficulty in identifying small graphics, the user can easily recognize the front appearance graphic 50 and the back appearance graphic 51 and easily identify the front and back of the diaper 1.

Also, with normal undergarments (underwear), the back panel is often larger than the front panel in order to cover the wearer's buttocks region. In view of this, it is desirable that the area of the back appearance graphic 51 is larger than the area of the front appearance graphic 50, as with the diaper 1 according to the first example and the second example. If the area of the back appearance graphic 51 is larger, the back portion 3 of the diaper 1 is more prominent than the front portion 2, and the user can be given the impression (illusion) that the back portion 3 is large. The user can therefore more intuitively identify the front and back of the diaper 1.

Note that the area of the front appearance graphic 50 and the area of the back appearance graphic 51 may be the same, as with the diaper 1 according to the third example. Also, note that the area of the front appearance graphic 50 may be larger than the area of the back appearance graphic 51, as with the diaper 1 according to the fourth example.

Also, it is preferable that, in the state where the front portion 2 and the back portion 3 are overlaid on each other in the front-back direction (thickness direction) of the diaper 1, an upper end 51a (e.g., see FIG. 9) of the back appearance graphic 51 is located higher than an upper end 50a (e.g., see FIG. 8) of the front appearance graphic 50, and a lower end 51b of the back appearance graphic 51 is located lower than a lower end 50b of the front appearance graphic 50.

In other words, it is desirable that the vertical length of the back appearance graphic 51 is larger than the vertical length of the front appearance graphic 50. According to this configuration, the back portion 3 (back appearance graphic 51) of the diaper 1 is more prominent, and the user can be given the impression (illusion) that the back portion 3 is large. The user can therefore more intuitively identify the front and back of the diaper 1.

Also, it is desirable that at least a portion of the front appearance graphic 50 is arranged in the front waist region 4, and at least a portion of the back appearance graphic 51 is arranged in the back waist region 6, as with the diaper 1 according to the first to fourth examples. According to this configuration, the front appearance graphic 50 can be arranged in the central portion of the front portion 2 of the diaper 1, and the back appearance graphic 51 can be arranged in the back portion 3. The user is therefore more likely to recognize the front appearance graphic 50 and the back appearance graphic 51. The design of the diaper 1 also improves. However, there is no limitation to this, and the front appearance graphic 50 and the back appearance graphic 51 may be arranged in only the crotch region 5.

In the case of the pull-on disposable diaper 1, the elastic strings 23 and 33 (stretchable member) that stretch and contract in the lateral direction are arranged in the front waist region 4 and the back waist region 6. Only the stretchable portions (portions that exhibit stretchability) of the elastic strings 23 and 33 are shown in the figures. It is preferable that, as shown in FIGS. 8 and 9, the stretchable portions are not provided in the lateral central portion (i.e., the portion overlapped with the absorbent core 11A) of at least some of the elastic strings that are overlapped with the absorbent main body 10 (absorbent core 11A) in the vertical direction (elastic strings 23b and 33b). According to this configuration, it is possible to ensure the flatness of the absorbent core 11A.

In this case, it is preferable that at least a portion of the front appearance graphic 50 arranged in the front waist region 4 and at least a portion of the back appearance graphic 51 arranged in the back waist region 6 are overlapped with the absorbent core 11A. According to this configuration, the front appearance graphic 50 and the back appearance graphic 51 are arranged in flat portions of the diaper 1, and the visibility of the front appearance graphic 50 and the back appearance graphic 51 improves.

Furthermore, it is preferable that at least a portion of the front appearance graphic 50 arranged in the front waist region 4 and at least a portion of the back appearance graphic 51 arranged in the back waist region 6 are not overlapped with the stretchable portions of the elastic strings 23 and 33. According to this configuration, it is possible to suppress the case where the front appearance graphic 50 and the back appearance graphic 51 contract due to the elastic string 23 and 33, thus improving the visibility of the front appearance graphic 50 and the back appearance graphic 51.

Note that a configuration is possible in which, as with the diaper 1 according to the first example and the second example, the upper end portion of the back appearance graphic 51 is overlapped with the stretchable portions of the elastic strings 33 (i.e., is overlapped with elastic strings 33a that do not include non-stretchable portions in the lateral central portion), and the upper end portion of the front appearance graphic 50 is not overlapped with the stretchable portions of the elastic strings 23 (i.e., is not overlapped with elastic strings 23a that do not include non-stretchable portions in the lateral central portion). In other words, it is preferable that the length of the stretchable portions of the elastic strings 23 that are overlapped with the front appearance graphic 50 is smaller than the length of the stretchable portions of the elastic strings 33 that are overlapped with the back appearance graphic 51.

According to this configuration, the size of the back appearance graphic 51 can be increased (the vertical length can be increased). Also, the front appearance graphic 50 shows the ears, eyes, nose, and the like of the character, which are relatively small. For this reason, by arranging the front appearance graphic 50 so as to be overlapped with the stretchable portions of the elastic strings 23 as little as possible, it is possible to prevent the small parts of the character shown by the front appearance graphic 50 from contracting and being difficult to recognize. On the other hand, the back appearance graphic 51 shows the buttocks, tail, and the like of the character, which are relatively large. For this reason, even if the back appearance graphic 51 contracts a certain extent due to the elastic strings 33a, the back appearance graphic 51 can still be recognized.

Also, it is preferable that the back appearance graphic 51 is arranged extending from the back waist region 6 into the crotch region 5, as with the diaper 1 according to the first example, the second example, and the third example. If the back appearance graphic 51 is large enough to extend from the back waist region 6 into the crotch region 5, then even if the diaper 1 is replaced in a dimly-lit place or the diaper 1 is replaced by an elderly person, the user is likely to recognize the back appearance graphic 51 and likely to identify the front and back of the diaper 1. However, as with the diaper 1 according to the fourth example, the back appearance graphic 51 is not required to extend into the crotch region 5.

Furthermore, it is preferable that, as shown in FIGS. 9 and 11, the area of the back appearance graphic 51 arranged in the back waist region 6 is larger than the area of the back appearance graphic 51 arranged in the crotch region 5. According to this configuration, the user is likely to focus on the back waist region 6, and is likely to recognize that the elastic strings 33 are arranged in the back waist region 6. Accordingly, the user can first identify the front and back of the diaper 1, and then spread the back waist region 6 and the front waist region 4 with their hands while putting the diaper 1 on an infant. Also, the back waist region 6 can give the user the impression of fitting well.

In another example, the back appearance graphic 51 shown in FIG. 11 has a portion (contracting portion) that is arranged in the back waist region 6 and is overlapped with the elastic strings 33, a portion (non-contracting portion) that is arranged in the back waist region 6 and is not overlapped with the elastic strings 33, and a portion that is not arranged in the back waist region 6. For this reason, even if the back appearance graphic 51 of a cat is printed entirely with the same color and same tone, a color gradation appears in the back appearance graphic 51, and the design of the diaper 1 improves.

Also, it is preferable that the front appearance graphic 50 is also arranged extending from the front waist region 4 into the crotch region 5, as with the diaper 1 according to the second example, the third example, and the fourth example. If the front appearance graphic 50 is large enough to extend from the front waist region 4 into the crotch region 5, then even if the diaper 1 is replaced in a dimly-lit place or the diaper 1 is replaced by an elderly person, the user is likely to recognize the front appearance graphic 50 and likely to identify the front and back of the diaper 1. However, as with the diaper 1 according to the first example, the front appearance graphic 50 is not required to extend into the crotch region 5.

Furthermore, it is preferable that, as with the diaper 1 according to the first example and the second example, a value obtained by dividing the area of the portion of the back appearance graphic 51 arranged in the crotch region 5 by the total area of the back appearance graphic 51 is greater than a value obtained by dividing the area of the portion of the front appearance graphic 50 arranged in the crotch region 5 by the total area of the front appearance graphic 50. In other words, it is preferable that the proportion of the back appearance graphic 51 that extends into the crotch region 5 is greater than the proportion of the front appearance graphic 50 that extends into the crotch region 5.

According to this configuration, the back portion 3 (back appearance graphic 51) of the diaper 1 is more prominent, and the user can be given the impression (illusion) that the back portion 3 is large. The user can therefore more intuitively identify the front and back of the diaper 1. Also, it is possible to ensure that the front portion 2 has a space for arranging a front indication text 52 (e.g., "front"), which indicates that it is the portion coming into contact with the wearer's front side, as shown in FIGS. 8 and 9. However, there is no limitation to this, and the proportion of the front appearance graphic 50 that extends into the crotch region 5 may be greater than the proportion of the back appearance graphic 51 that extends into the crotch region 5. Also, the proportion extending into the crotch region 5 may be the same for the front appearance graphic 50 and the back appearance graphic 51.

A comparison of the areas of the graphics, a comparison of the positions of the graphics in the vertical direction, and a comparison of the lengths of the stretchable portions of the stretchable members overlapped with the graphics have been described above, and it is preferable that these comparisons are performed when the diaper 1 is in the stretched state (the diaper 1 is stretched such that wrinkles formed therein are substantially no longer visible), as shown in FIG. 2 for example. Also, the comparison of the areas of the graphics can be performed using a known method. For example, the areas may be compared visually, and it is possible to use area calculation software to calculate the areas based on captured images of the graphics and then compare the calculated areas.

Also, in general diapers that have character graphics, graphics that show the front appearance of the character are arranged on both the front portion and the back portion. For this reason, a user who sees the back appearance graphic 51 of the diaper 1 of the present embodiment for the first time is likely to immediately identify the front and back of the diaper 1. On the other hand, a user who sees the front appearance graphic 50 for the first time is likely to identify the front and back of the diaper 1 by checking the back appearance graphic 51. In view of this, it is preferable that the front indication text 52 (e.g., "front") is arranged on the front portion 2 as with the diaper 1 according to the first example, the second example, and the fourth example.

According to this configuration, a user who sees the front appearance graphic 50 for the first time can immediately identify the front and back of the diaper 1. Also, both when the wearer puts on the diaper 1 and when a caregiver puts the diaper 1 on an infant, the diaper 1 is often put on while looking at the front portion 2. For this reason, arranging the front indication text 52 on the front portion 2 makes it possible for the wearer or the caregiver to check that the front and back of the diaper 1 are correct, and the diaper 1 can be worn and put on with confidence.

In contrast, a configuration is possible in which the back portion 3 does not have back indication text (e.g., "back") indicating that it is the portion coming into contact with the wearer's back, as with the diaper 1 according to the first example and the second example. According to this configuration, the size of the back appearance graphic 51 can be increased. Also, as described above, a user who sees the back appearance graphic 51 for the first time is likely to intuitively identify the front and back of the diaper 1 even without the back indication text.

However, there is no limitation to the above configuration. A configuration is possible in which neither the front indication text nor the back indication text are provided, as with the diaper 1 according to the third example. Conversely, both the front indication text 52 and the back indication text 53 may be provided, as with the diaper 1 according to the fourth example. Also, the diaper may have a configuration in which the front indication text is not provided, and only the back indication text is provided (not shown).

Also, in the case of the pull-on disposable diaper 1, stretchable members (the elastic strings 23 and 33 and the stretchy sheet 24) are arranged in the front waist region 4 and the back waist region 6. For this reason, it is preferable that the front indication text 52 and the back indication text 53 are arranged in the crotch region 5. According to this configuration, it is possible to prevent the case where the front indication text 52 and the back indication text 53 contract and become difficult to recognize due to the stretchable members.

Also, in the diaper 1 of the present embodiment the front appearance graphic 50 is relatively large. Specifically, it is preferable that the vertical length of the front appearance graphic 50 is greater than or equal to 30% of the vertical length of a print region of the front portion 2 (in the present embodiment, the portion of the back sheet 13 that is arranged in the front portion 2). It is preferable that, in comparison with the relatively large front appearance graphic 50, the area of the front indication text 52 is in a range of 15% to 25% inclusive of the area of the front appearance graphic 50. Also, it is preferable that the area of the front indication text 52 is in a range of 2% to 4% inclusive of the print region of the front portion 2 (in the present embodiment, the portion of the back sheet 13 that is arranged in the front portion 2). Note that the area of the front indication text 52 includes not only the text portion, but also a graphic that surrounds the text (e.g., the oval-like shape in FIG. 8).

According to this configuration, it is possible to suppress the case where the front indication text 52 is too small and not likely to be recognized by the user, and It is also possible to suppress the case where the front indication text 52 is too large and degrades the design of the diaper 1. In this way, it is desirable that the front appearance graphic 50 and the front indication text 52 are formed as large as possible without degrading the design. According to this configuration, both when the diaper 1 is replaced in a dimly-lit place and when the diaper 1 is replaced by an elderly person, the user is likely to recognize the front appearance graphic 50 and the front indication text 52. Also, even if the front indication text 52 is relatively large, the front appearance graphic 50 is even larger, and the user is likely to focus on the front appearance graphic 50. This therefore makes it possible to suppress a degradation in design caused by the front indication text 52 being large.

Also, in the case where the front indication text 52 is arranged on the front portion 2 and the back indication text is not arranged on the back portion 3 as with the diaper 1 according to the first example and the second example, it is desirable that, as shown in FIG. 9 for example, the front indication text 52 and the back appearance graphic 51 are overlapped in the front-back direction of the diaper 1. Furthermore, the area of the back appearance graphic 51 may be larger than the total of the area of the front appearance graphic 50 and the area of the front indication text 52. Due to forming the back appearance graphic 51 this large, the user can more easily recognize the back appearance graphic 50 both when the diaper 1 is replaced in a dimly-lit place and when the diaper 1 is replaced by an elderly person. Also, the back portion 3 of the diaper 1 is more prominent, and the user can more intuitively identify the front and back of the diaper 1.

Also, as with the diaper 1 according to the second example, graphics 54 that are different from the front appearance graphic 50 and the back appearance graphic 51 may be provided. In this case, it is desirable that the areas of the front appearance graphic 50 and the back appearance graphic 51 are greater than the area of the front indication text 52, and that the area of the front indication text 52 is greater than the areas of the other graphics 54 (e.g., the areas of stars or the areas of hearts). According to this configuration, the front appearance graphic 50 and the back appearance graphic 51 are most easily recognized by the user, and it is possible to suppress a degradation in design caused by the front indication text 52 being large. Also, the front indication text 52 is not too small, thus making it possible to suppress the case where the front indication text 52 is not easily recognized by the user.

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof.

### [Reference Signs List]

1 pull-on disposable diaper (absorbent article),
2 front portion, 3 back portion,
4 front waist region, 5 crotch region, 6 back waist region,
10 absorbent main body, 11 absorbent body,
11A absorbent core, 11C narrow portion,
12 top sheet, 13 back sheet, 14 exterior sheet,
20 front belt member, 30 back belt member,
21, 31 skin-side sheet, 22, 32 non-skin-side sheet,
23, 33 elastic string, 24 stretchy sheet,
34 sweat absorbing sheet,
40 stool containing pocket (pocket), 41 upper end member, 42 lower end member,
43 upper end portion (waist contact portion), 47 post-processing tape,
50 front appearance graphic, 51 back appearance graphic,
52 front indication text (text), 53 back indication text (text)

## Claims

1. An absorbent article (1) having a vertical direction, a lateral direction, and a front-back direction that intersect each other,
the absorbent article (1) comprising:
a front portion (2) and a back portion (3) in the front-back direction,
the back portion (3) including a portion having a different function from the front portion (2);
a front appearance graphic (50),
the front appearance graphic (50) showing at least a portion of a front appearance of a character and arranged on the front portion (2); and
a back appearance graphic (51),
the back appearance graphic (51) showing at least a portion of a back appearance of the character and arranged on the back portion (3),
wherein
the absorbent article (1) is an underpants-shaped absorbent article (1),
the underpants-shaped absorbent article (1) including a front waist region (4), a back waist region (6), and a crotch region (5),
two lateral side end portions of the front waist region (4) being joined to two lateral side end portions of the back waist region (6),
the front waist region (4) and the back waist region (6) each including a stretchable member capable of stretching and contracting in the lateral direction,
wherein
a length of a stretchable portion of a stretchable member that is overlapped with the front appearance graphic (50) is smaller than a length of a stretchable portion of a stretchable member that is overlapped with the back appearance graphic (51),
the absorbent article (1) includes an absorbent main body (10),
and the front appearance graphic (50) and the back appearance graphic (51) are printed on a non-skin-side surface of a liquid-impermeable back sheet (13) of the absorbent main body (10).

2. The absorbent article (1) according to claim 1, wherein
the front appearance graphic (50) is a graphic showing at least a portion of an upper body of the character, and
the back appearance graphic (51) is a graphic showing at least a portion of a lower body of the character.

3. The absorbent article (1) according to claim 1 or 2, wherein
an area of the back appearance graphic (51) is larger than an area of the front appearance graphic (50).

4. The absorbent article (1) according to any one of claims 1 to 3, wherein
an upper end (51a) of the back appearance graphic (51) is located higher than an upper end (50a) of the front appearance graphic (50), and
a lower end (51b) of the back appearance graphic (51) is located lower than a lower end (50b) of the front appearance graphic (50).

5. The absorbent article (1) according to any one of claims 1 to 4, wherein
the front portion (2) has text (52) indicating that it is a portion that is to come into contact with a front side of a wearer.

6. The absorbent article (1) according to claim 5, wherein
the back portion (3) does not have text indicating that it is a portion that is to come into contact with a back side of a wearer.

7. The absorbent article (1) according to any preceding claim, wherein
text (52, 53) indicating that it is a portion that is to come into contact with either one of a front side of a wearer and a back side of a wearer is arranged in the crotch region (5).

8. The absorbent article (1) according to any preceding claim, wherein
the back appearance graphic (51) is arranged extending from the back waist region (6) into the crotch region (5).

9. The absorbent article (1) according to claim 8, wherein
an area of a portion of the back appearance graphic (51) arranged in the back waist region (6) is larger than an area of a portion of the back appearance graphic (51) arranged in the crotch region (5).

10. The absorbent article (1) according to preceding claim, wherein
the front appearance graphic (50) is arranged extending from the front waist region (4) into the crotch region (5).

11. The absorbent article (1) according to claim 9 or 10, wherein
a value obtained by dividing an area of a portion of the back appearance graphic (51) arranged in the crotch region (5) by a total area of the back appearance graphic (51)
is greater than
a value obtained by dividing an area of a portion of the front appearance graphic (50) arranged in the crotch region (5) by a total area of the front appearance graphic (50).

12. The absorbent article (1) according to preceding claim, wherein
post-processing tape is not provided in a lateral central portion of the back waist region (6).

13. The absorbent article (1) according to any preceding claim, wherein
the front waist region (4) is provided with a stretchy sheet (24) as the stretchable member, and
the back waist region (6) is not provided with the stretchy sheet.

14. The absorbent article (1) according to any preceding claim, wherein
the absorbent article (1) further comprises a front waist region (4), a back waist region (6), and a crotch region (5),
the back waist region is provided with a pocket (40),
the pocket being located on a non-skin side with respect to a waist contact portion (43) extending in the lateral direction,
the pocket facing the waist contact portion in the front-back direction at a lateral central portion,
the pocket being capable of opening toward the crotch region (5), and
the front waist region (4) is not provided with the pocket.

15. The absorbent article (1) according to any preceding claim, wherein
the absorbent article (1) further comprises an absorbent core (11),
the absorbent core includes a narrow portion (11C),
the narrow portion having a lateral width that is smaller than a lateral width of an upper end (11a) of the absorbent core, and
a vertical length (L1) of a portion of the narrow portion arranged in the front portion (2) is larger than a vertical length (L2) of a portion of the narrow portion arranged in the back portion (3).

16. The absorbent article (1) according to any preceding claim, wherein
the back portion (3) is provided with a sweat absorbing sheet (34) containing hydrophilic fibers, on a skin-side surface of the back portion (3), and
the front portion (2) is not provided with the sweat absorbing sheet.

## Patentansprüche

1. Absorbierender Artikel (1), der eine vertikale Richtung, eine laterale Richtung und eine Vorn-Hinten-Richtung aufweist, die einander schneiden,
wobei der absorbierende Artikel (1) Folgendes umfasst:
ein vorderes Teil (2) und ein hinteres Teil (3) in der Vorn-Hinten-Richtung,
wobei das hintere Teil (3) einen Teil einschließt, der eine andere Funktion als das vordere Teil (2) aufweist;
eine vorn erscheinende Abbildung (50),
wobei die vorn erscheinende Abbildung (50) zumindest einen Teil eines vorderen Erscheinungsbilds einer Figur zeigt und auf dem vorderen Teil (2) angeordnet ist; und
eine hinten erscheinende Abbildung (51),
wobei die hinten erscheinende Abbildung (51) zumindest einen Teil eines hinteren Erscheinungsbilds der Figur zeigt und auf dem hinteren Teil (3) angeordnet ist, wobei
der absorbierende Artikel (1) ein Unterhosen-förmiger absorbierender Artikel (1) ist,
wobei der Unterhosen-förmige absorbierende Artikel (1) einen vorderen Taillenbereich (4), einen hinteren Taillenbereich (6) und einen Schrittbereich (5) einschließt,
zwei laterale Seitenendteile des vorderen Taillenbereichs (4) mit zwei lateralen Seitenendteilen des hinteren Taillenbereichs (6) verbunden sind,
der vordere Taillenbereich (4) und der hintere Taillenbereich (6) jeweils ein dehnbares Element einschließen, das sich in der lateralen Richtung dehnen und zusammenziehen kann,
wobei
eine Länge eines dehnbaren Teils eines dehnbaren Elements, die mit der vorn erscheinenden Abbildung (50) überlappt, kleiner ist als eine Länge eines dehnbaren Teils eines dehnbaren Elements, die mit der hinten erscheinenden Abbildung (51) überlappt,
der absorbierende Artikel (1) einen absorbierenden Hauptkörper (10) einschließt und die vorn erscheinende Abbildung (50) und die hinten erscheinende Abbildung (51) auf eine Nicht-Hautseitenoberfläche einer flüssigkeitsundurchlässigen hinteren Lage (13) des absorbierenden Hauptkörpers (10) gedruckt sind.

2. Absorbierender Artikel (1) nach Anspruch 1, wobei
die vorn erscheinende Abbildung (50) eine Abbildung ist, die zumindest einen Teil eines oberen Körpers der Figur zeigt, und
die hinten erscheinende Abbildung (51) eine Abbildung ist, die zumindest einen Teil eines unteren Körpers der Figur zeigt.

3. Absorbierender Artikel (1) nach Anspruch 1 oder 2, wobei
eine Fläche der hinten erscheinenden Abbildung (51) größer ist als eine Fläche der vorn erscheinenden Abbildung (50).

4. Absorbierender Artikel (1) nach einem der Ansprüche 1 bis 3, wobei
ein oberes Ende (51a) der hinten erscheinenden Abbildung (51) höher gelegen ist als ein oberes Ende (50a) der vorn erscheinenden Abbildung (50) und
ein unteres Ende (51b) der hinten erscheinenden Abbildung (51) niedriger gelegen ist als ein unteres Ende (50b) der vorn erscheinenden Abbildung (50).

5. Absorbierender Artikel (1) nach einem der Ansprüche 1 bis 4, wobei
der vordere Teil (2) Schrift (52) aufweist, die anzeigt, dass es ein Teil ist, der mit einer vorderen Seite eines Trägers in Kontakt zu bringen ist.

6. Absorbierender Artikel (1) nach Anspruch 5, wobei
der hintere Teil (3) keine Schrift aufweist, was anzeigt, dass es ein Teil ist, der mit einer hinteren Seite eines Trägers in Kontakt zu bringen ist.

7. Absorbierender Artikel (1) nach einem vorstehenden Anspruch, wobei
Schrift (52, 53), die anzeigt, dass es ein Teil ist, der mit einer einer vorderen Seite eines Trägers oder einer hinteren Seite eines Trägers in Kontakt zu bringen ist, in dem Schrittbereich (5) angeordnet ist.

8. Absorbierender Artikel (1) nach einem vorstehenden Anspruch, wobei
die hinten erscheinende Abbildung (51) angeordnet ist, dass sie sich von dem hinteren Taillenbereich (6) in den Schrittbereich (5) erstreckt.

9. Absorbierender Artikel (1) nach Anspruch 8, wobei
eine Fläche eines Teils der hinten erscheinenden Abbildung (51), die in dem hinteren Taillenbereich (6) angeordnet ist, größer ist als eine Fläche eines Teils der hinten erscheinenden Abbildung (51), die in dem Schrittbereich (5) angeordnet ist.

10. Absorbierender Artikel (1) nach vorstehendem Anspruch, wobei
die vorn erscheinende Abbildung (50) angeordnet ist, dass sie sich von dem vorderen Taillenbereich (4) in den Schrittbereich (5) erstreckt.

11. Absorbierender Artikel (1) nach Anspruch 9 oder 10, wobei
ein Wert, der durch Dividieren einer Fläche eines Teils der hinten erscheinenden Abbildung (51), die in dem Schrittbereich (5) angeordnet ist, durch eine Gesamtfläche der hinten erscheinenden Abbildung (51) erhalten wird,
größer ist als
ein Wert, der durch Dividieren einer Fläche eines Teils der vorn erscheinenden Abbildung (50), die in dem Schrittbereich (5) angeordnet ist, durch eine Gesamtfläche der vorn erscheinenden Abbildung (50) erhalten wird.

12. Absorbierender Artikel (1) nach vorstehendem Anspruch, wobei
Nachverarbeitungsband in einem lateralen mittleren Teil des hinteren Taillenbereichs (6) nicht bereitgestellt ist.

13. Absorbierender Artikel (1) nach einem vorstehenden Anspruch, wobei
der vordere Taillenbereich (4) mit einer dehnbaren Lage (24) als das dehnbare Element bereitgestellt ist und
der hintere Taillenbereich (6) nicht mit der dehnbaren Lage bereitgestellt ist.

14. Absorbierender Artikel (1) nach einem vorstehenden Anspruch, wobei
der absorbierende Artikel (1) weiter einen vorderen Taillenbereich (4), einen hinteren Taillenbereich (6) und einen Schrittbereich (5) umfasst,
der hintere Taillenbereich mit einer Tasche (40) bereitgestellt ist,
wobei sich die Tasche auf einer Nicht-Hautseite in Bezug auf einen Taillenkontaktteil (43), der sich in der lateralen Richtung erstreckt, befindet,
die Tasche dem Taillenkontaktteil in der Von-Hinten-Richtung an einem lateralen mittleren Teil zugewandt ist,
die Tasche in Richtung des Schrittbereichs (5) geöffnet werden kann, und der vordere Taillenbereich (4) nicht mit der Tasche bereitgestellt ist.

15. Absorbierender Artikel (1) nach einem vorstehenden Anspruch, wobei
der absorbierende Artikel (1) weiter einen Saugkern (11) umfasst,
der Saugkern einen schmalen Teil (11C) einschließt,
wobei der schmale Teil eine laterale Breite aufweist, die kleiner ist als eine laterale Breite eines oberen Endes (11a) des Saugkerns, und
eine vertikale Länge (L1) eines Teils des schmalen Teils, der in dem vorderen Teil (2) angeordnet ist, größer ist als eine vertikale Länge (L2) eines Teils des schmalen Teils, der in dem hinteren Teil (3) angeordnet ist.

16. Absorbierender Artikel (1) nach einem vorstehenden Anspruch, wobei
der hintere Teil (3) mit einer schweißabsorbierenden Lage (34), die hydrophile Fasern enthält, auf einer Hautseitenoberfläche des hinteren Teils (3) bereitgestellt ist und
der vordere Teil (2) nicht mit der schweißabsorbierenden Lage bereitgestellt ist.

## Revendications

1. Article absorbant (1) ayant une direction verticale, une direction latérale et une direction avant-arrière qui se croisent les unes les autres,
l'article absorbant (1), comportant :
une partie avant (2) et une partie arrière (3) dans la direction avant-arrière,
la partie arrière (3) comprenant une partie ayant une fonction différente de celle de la partie avant (2) ;
une représentation graphique côté apparaissant devant (50),
la représentation graphique côté apparaissant devant (50) représentant au moins une partie d'un côté apparaissant devant d'un personnage et étant disposée sur la partie avant (2) ; et
une représentation graphique côté apparaissant derrière (51),
la représentation graphique côté apparaissant derrière (51) représentant au moins une partie d'un côté apparaissant derrière du personnage et étant disposée sur la partie arrière (3),
dans lequel
l'article absorbant (1) est un article absorbant en forme de culotte (1),
l'article absorbant en forme de culotte (1) comprenant une région avant au niveau de la taille (4), une région arrière au niveau de la taille (6) et une région au niveau de l'entrejambe (5),
deux parties d'extrémité côté latéral de la région avant au niveau de la taille (4) étant jointes à deux parties d'extrémité côté latéral de la région arrière au niveau de la taille (6),
la région avant au niveau de la taille (4) et la région arrière au niveau de la taille (6) comprenant chacune un élément extensible en mesure de s'étirer et de se contracter dans la direction latérale,
dans lequel
une longueur d'une partie extensible d'un élément extensible qui est recouvert par la représentation graphique côté apparaissant devant (50) est inférieure à une longueur d'une partie extensible d'un élément extensible qui est recouvert par la représentation graphique côté apparaissant derrière (51),
l'article absorbant (1) comprend un corps principal absorbant (10),
et la représentation graphique côté apparaissant devant (50) et la représentation graphique côté apparaissant derrière (51) sont imprimées sur une surface côté non orienté vers la peau d'une feuille arrière imperméable aux liquides (13) du corps principal absorbant (10).

2. Article absorbant (1) selon la revendication 1, dans lequel
la représentation graphique côté apparaissant devant (50) est une représentation graphique qui représente au moins une partie d'un corps supérieur du personnage, et
la représentation graphique côté apparaissant derrière (51) est une représentation graphique qui représente au moins une partie d'un corps inférieur du personnage.

3. Article absorbant (1) selon la revendication 1 ou la revendication 2, dans lequel
une zone de la représentation graphique côté apparaissant derrière (51) est supérieure à une zone de la représentation graphique côté apparaissant devant (50).

4. Article absorbant (1) selon l'une quelconque des revendications 1 à 3, dans lequel
une extrémité supérieure (51a) de la représentation graphique côté apparaissant derrière (51) est située plus haut par rapport à une extrémité supérieure (50a) de la représentation graphique côté apparaissant devant (50), et
une extrémité inférieure (51b) de la représentation graphique côté apparaissant derrière (51) est située plus bas par rapport à une extrémité inférieure (50b) de la représentation graphique côté apparaissant devant (50).

5. Article absorbant (1) selon l'une quelconque des revendications 1 à 4, dans lequel
la partie avant (2) contient du texte (52) indiquant qu'il s'agit d'une partie qui est destinée à entrer en contact avec un côté avant d'un utilisateur.

6. Article absorbant (1) selon la revendication 5, dans lequel la partie arrière (3) ne contient pas de texte indiquant qu'il s'agit d'une partie qui est destinée à entrer en contact avec un côté arrière d'un utilisateur.

7. Article absorbant (1) selon l'une quelconque des revendications précédentes, dans lequel
du texte (52, 53) indiquant qu'il s'agit d'une partie qui est destinée à entrer en contact avec l'un ou l'autre parmi un côté avant d'un utilisateur et un côté arrière d'un utilisateur est agencé dans la région au niveau de l'entrejambe (5).

8. Article absorbant (1) selon l'une quelconque des revendications précédentes, dans lequel
la représentation graphique côté apparaissant derrière (51) est agencée de manière à s'étendre depuis la région arrière au niveau de la taille (6) jusque dans la région au niveau de l'entrejambe (5).

9. Article absorbant (1) selon la revendication 8, dans lequel
une zone d'une partie de la représentation graphique côté apparaissant derrière (51) agencée dans la région arrière au niveau de la taille (6) est supérieure à une zone d'une partie de la représentation graphique côté apparaissant derrière (51) agencée dans la région au niveau de l'entrejambe (5).

10. Article absorbant (1) selon l'une quelconque des revendications précédentes, dans lequel
la représentation graphique côté apparaissant devant (50) est agencée de manière à s'étendre depuis la région avant au niveau de la taille (4) jusque dans la région au niveau de l'entrejambe (5).

11. Article absorbant (1) selon la revendication 9 ou la revendication 10, dans lequel
une valeur obtenue en divisant une zone d'une partie de la représentation graphique côté apparaissant derrière (51) agencée dans la région au niveau de l'entrejambe (5) par une zone totale de la représentation graphique côté apparaissant derrière (51)
est supérieure à
une valeur obtenue en divisant une zone d'une partie de la représentation graphique côté apparaissant devant (50) agencée dans la région au niveau de l'entrejambe (5) par une zone totale de la représentation graphique côté apparaissant devant (50).

12. Article absorbant (1) selon l'une quelconque des revendications précédentes, dans lequel
une bande de post-traitement n'est pas mise en œuvre dans une partie centrale latérale de la région arrière au niveau de la taille (6).

13. Article absorbant (1) selon l'une quelconque des revendications précédentes, dans lequel
la région avant au niveau de la taille (4) est dotée d'une feuille extensible (24) en tant qu'élément extensible, et
la région arrière au niveau de la taille (6) n'est pas dotée de la feuille extensible.

14. Article absorbant (1) selon l'une quelconque des revendications précédentes, dans lequel
l'article absorbant (1) comporte par ailleurs une région avant au niveau de la taille (4), une région arrière au niveau de la taille (6) et une région au niveau de l'entrejambe (5),
la région arrière au niveau de la taille est dotée d'une poche (40),
la poche étant située sur un côté non orienté vers la peau par rapport à une partie de contact au niveau de la taille (43) s'étendant dans la direction latérale,
la poche étant orientée vers la partie de contact au niveau de la taille dans la direction avant-arrière au niveau d'une partie centrale latérale,
la poche étant en mesure de s'ouvrir vers la région au niveau de l'entrejambe (5), et
la région avant au niveau de la taille (4) n'est pas dotée d'une poche.

15. Article absorbant (1) selon l'une quelconque des revendications précédentes, dans lequel
l'article absorbant (1) comporte par ailleurs une partie centrale absorbante (11),
la partie centrale absorbante comprend une partie étroite (11C),
la partie étroite ayant une largeur latérale inférieure à une largeur latérale d'une extrémité supérieure (11a) de la partie centrale absorbante, et
une longueur verticale (L1) d'une partie de la partie étroite agencée dans la partie avant (2) est supérieure à une longueur verticale (L2) d'une partie de la partie étroite agencée dans la partie arrière (3).

16. Article absorbant (1) selon l'une quelconque des revendications précédentes, dans lequel
la partie arrière (3) est dotée d'une feuille absorbant la transpiration (34) contenant des fibres hydrophiles, sur une surface côté orienté vers la peau de la partie arrière (3), et
la partie avant (2) n'est pas dotée de la feuille absorbant la transpiration.
